## Europäisches Patentamt
## European Patent Office
## Office européen des brevets

(19)

(11) Numéro de publication: **0 171 312**
**B1**

(12)

# FASCICULE DE BREVET EUROPÉEN

(45) Date de publication du fascicule du brevet:
04.02.87

(21) Numéro de dépôt: **85401358.8**

(22) Date de dépôt: **04.07.85**

(51) Int. Cl.⁴: **C 07 C 149/06,** B 01 J 27/047, B 01 J 27/04

---

(54) Procédé de fabrication de méthylmercaptan à partir des oxydes de carbone.

---

(30) Priorité: **10.07.84 FR 8410928**

(43) Date de publication de la demande:
**12.02.86 Bulletin 86/7**

(45) Mention de la délivrance du brevet:
**04.02.87 Bulletin 87/6**

(84) Etats contractants désignés:
**DE GB NL**

(56) Documents cité:
**EP-A-0 104 507**
**US-A-3 070 632**
**US-A-4 410 731**

(73) Titulaire: **SOCIETE NATIONALE ELF AQUITAINE (PRODUCTION), Tour Elf 2, Place de la Coupole La Défense 6, F-92400 Courbevoie (FR)**

(72) Inventeur: **Boulinguiez, Martine, Ger, F-64530 Pontacq (FR)**
Inventeur: **Barrault, Joel, La Brassaise, F-86240 Liguge (FR)**
Inventeur: **Forquy, Christian, Cité des Mimosas Cidex 5, Artiguelouve, F-64230 Lescar (FR)**

(74) Mandataire: **Kohn, Armand, 5 Avenue Foch, F-92380 Garches (FR)**

**Description**

La présente invention est un perfectionnement à la production de méthyl-mercaptan à partir de mélanges gazeux renfermant au moins un des oxydes de carbone, de l'hydrogène sulfuré et de l'hydrogène. Elle permet l'obtention de ce produit avec des productivités horaires considérablement accrues par rapport à celles qu'il était possible d'atteindre dans les procédés de la technique antérieure.

Etant donné l'intérêt industriel du méthyl-mercaptan, en particulier comme matière première de fabrication de différents composés utiles à l'agriculture et à l'élevage, on a cherché à obtenir ce corps aussi économiquement que possible. Le procédé, qui consiste à chauffer des gaz composés de CO ou/et $CO_2$, $H_2S$ et $H_2$ en présence d'un catalyseur à base de sulfure de métal lourd, a donné lieu à plusieurs publications; il pourrait, en effet, être très intéressant, vu le bon marché de ces matières premières; cependant, le taux de conversion trop faible de l'oxyde de carbone, pour des vitesses spatiales élevées des gaz, oblige à travailler avec des vitesses spatiales petites, qui conduisent à des faibles productivités en méthylmercaptan.

Ainsi, selon US 4 410 731, Tableaux 4 et 5, seule la vitesse spatiale de 5 l/l.h du CO ou $CO_2$ fournit des résultats acceptables: rendement de 89% sur CO et 49% sur $CO_2$. Avec 60 l/l.h le rendement ne dépasse plus 61% pour le CO et 24% pour le $CO_2$; à une vitesse spatiale de 180 on ne trouve que 49% sur CO (à noter que la littérature anglo-saxonne désigne par "conversion" ce que l'on appelle rendement en France). De même, selon la demande OEB 0 184 507, Tableaux 2 et 3, pour des vitesses spatiales de 148 et 45, les rendements varient entre 21 et 40%. Donc, à l'échelle industrielle, il faut recycler les gaz n'ayant pas réagi, comme d'ailleurs suggéré dans l'US précité, col. 6, lignes 1-3. Mais alors on peut constater que les gaz recyclés conduisent à des conversions encore beaucoup plus faibles, ce qui n'est pas signalé dans la littérature où les auteurs donnent les résultats d'une seule passe sur le catalyseur.

Il apparaît que, pour remédier aux faiblesses susindiquées du procédé connu, on ait seulement cherché, jusqu'à présent, à trouver un catalyseur idoine. C'est ainsi que l'USP 3 070 632 propose l'adjonction d'une amine au catalyseur de sulfure métallique, l'USP 4 410 731 améliore quelque peu la conversion par l'emploi d'un promoteur, un sulfure de métal alcalin, et la demande européenne 0 104 507 par l'utilisation de sulfure de manganèse. Toutefois, aucun de ces moyens ne permet d'améliorer la réutilisation des gaz recyclés. C'est l'étude de cette question qui a donné naissance à la présente invention. Celle-ci résulte de la découverte que les difficultés de réutilisation des gaz, qui n'ont pas réagi, sont dues à l'inhibition de la réaction par l'eau que contiennent les gaz à la suite de leur premier passage sur le catalyseur. La séparation de cette eau permet d'utiliser les gaz recyclés avec un excellent rendement.

Suivant un premier trait de l'invention, le perfectionnement du procédé de fabrication de méthyl-mercaptan à partir des mélanges gazeux CO ou/et $CO_2$, $H_2S$ et $H_2$, sur un catalyseur de sulfure métallique, avec recyclage des gaz résiduels, est caractérisé en ce que les gaz à recycler sont débarrassés de l'eau qu'ils contiennent à la suite de la réaction au contact du catalyseur.

Le procédé perfectionné suivant l'invention comprend ainsi une opération de dessiccation des gaz résiduels, après la séparation du mercaptan produit, avant un second passage de ces gaz sur le catalyseur.

Dans une forme préférée de l'invention, ce second passage, et éventuellement les suivants, peut avoir lieu avec une vitesse spatiale élevée, notamment 50 à 2 000 litres par litre de catalyseur par heure, et plus particulièrement avec 580 à 1 800 l/l.h. Tout comme dans la technique antérieure, les vitesses spatiales s'entendent pour le CO, $CO_2$ ou $CO+CO_2$ passant dans le réacteur, sans compter les autres gaz présents, notamment $H_2S$ et $H_2$.

Le procédé de l'invention peut être réalisé avec des rapports (CO ou/et $CO_2$)/$H_2S$/$H_2$ du même ordre que ceux de la technique connue, c'est-à-dire environ 1/3/2 à 1/8/8. Cependant, l'invention présente l'avantage de ne pas nécessiter de forts excès d'$H_2S$ et de $H_2$ qui sont le plus souvent employés dans les procédés antérieurs; des rapports d'environ 1/3/3 à 1/4/6, et surtout proches de 1/4/4, suffisent.

Un autre avantage important de l'invention réside dans une forte amélioration de l'utilisation du $CO_2$ qui donne un rendement et une production horaire en méthyl-mercaptan bien supérieurs à ceux de l'art antérieur.

En ce qui concerne les conditions de température et de pression, elles peuvent être semblables à celles de la technique connue, notamment 250° à 350°C, de préférence 278° à 300°C, les pressions préférées étant de 12 à 50 bars et plus particulièrement environ 25 à 35 bars.

L'invention s'applique avec tous les catalyseurs connus et surtout avec du sulfure de tungstène ou celui de rhénium sur support d'alumine activée.

Ces catalyseurs préférés peuvent être préparés à la manière connue en soi, par sulfuration avec de l'$H_2S$ des oxydes ou sels alcalins correspondants. Il est avantageux d'effectuer arant la sulfuration une calcination vers 450°C en présence d'air.

Il est intéressant de constater qu'appliqué selon l'invention, le sulfure de tungstène, obtenu à partir d'un tungstate alcalin, donne seul, sans autres sulfures de métaux lourds, des conversions élevées, presque sans formation de méthane, alors que dans l'art antérieur il n'a servi que de promoteur.

Une caractéristique essentielle du procédé faisant l'objet de la présente invention est de pratiquer un recyclage des réactifs non consommés après une première passe des réactifs, en prenant soin d'éliminer l'eau formée et le méthylmercaptan déjà produit. Ceci peut être mis en oeuvre par des techniques connues, à savoir par séchage des gaz sur tamis moléculaire ou par fractionnement. De cette façon on évite l'emploi de vitesses spatiales très faibles qui n'étaient nécessaires qu'à cause d'une inhibition des réactions par l'eau formée.

La technique du procédé amélioré permet d'effectuer la réaction à partir du dioxyde de carbone à des

2

vitesses spatiales très supérieures à celles de la technique antérieure, même en tenant compte du recyclage.

En effet, la suite des réactions, par lesquelles le monoxyde de carbone est transformé, fait appel aux équations suivantes:

(1) $CO + H_2S \rightarrow COS + H_2$

(2) $COS + 3H_2 \rightarrow CH_3SH + H_2O$

En présence de catalyseurs d'oxydo-réduction tels que ceux qu'exige la réaction globale, l'eau est très rapidement éliminée du milieu par la réaction du gaz à l'eau:

(3) $CO + H_2O \leftrightarrows CO_2 + H_2$.

Si le dioxyde de carbone n'était pas transformé en méthylmercaptan par l'intermédiaire de l'oxysulfure de carbone, on aboutirait à des sélectivités identiques en méthylmercaptan et en dioxyde de carbone; dans ce cas, l'eau serait fatalement éliminée in situ dans le réacteur. Mais, contrairement aux hypothèses émises par les auteurs de l'art antérieur, le dioxyde de carbone est susceptible de réagir rapidement, en présence d'hydrogène sulfuré pour former de l'oxysulfure de carbone et de l'eau suivant l'équation:

(4) $CO_2 + H_2S \rightarrow COS + H_2O$.

La vitesse de formation du COS est en effet environ quatre fois plus élevée à partir du dioxyde de carbone qu'à partir du monoxyde de carbone. Mais, parallèlement, le dioxyde de carbone est transformé, avec une vitesse plus faible, en monoxyde de carbone par la réaction inverse de gaz à l'eau.

(3') $CO_2 + H_2 \leftrightarrows CO + H_2O$.

Le monoxyde de carbone est donc un sous produit fatal de la réaction entre le dioxyde de carbone, l'hydrogène sulfuré et l'hydrogène. On aboutit donc à un ensemble de réactions formant de l'eau en tant que sous-produit, ce qui a pour effet d'inhiber la réaction (4) de formation du COS-.

Pour cette raison, l'élimination de l'eau de réaction conduit à un système dans lequel le dioxyde de carbone, l'oxysulfure de carbone et le monoxyde de carbone se convertissent rapidement en méthyl-mercaptan, sans inhibition de la réaction de formation du COS-.

La prise en compte de ces considérations permet de réaliser la fabrication de méthyl-mercaptan à partir de dioxyde de carbone, d'hydrogène sulfuré et d'hydrogène avec une productivité compatible avec les exigences industrielles.

L'invention est illustrée par les exemples non limitatifs suivants.

**Exemple 1**

Préparation des catalyseurs

On prépare un catalyseur A en dissolvant 71,1 g d'oxyde de tungstène dans 1,5 1 d'une solution aqueuse de 41 g d'hydroxyde de potassium à 84% de KOH. On ajoute alors rapidement cette solution à 900 g d'alumine activée et l'on évapore à sec, puis on sèche 15 heures à 158°C. On obtient un catalyseur contenant 10% de tungstate de potassium stoechiométrique, déposé sur alumine activée.

On prépare un catalyseur B en dissolvant 36,0 g de perrhénate d'ammonium, $NH_4ReO_4$, dans 1,5 1 d'une solution aqueuse. On ajoute cette solution à 970 g d'alumine activée. On évapore l'eau à pression atmosphérique et l'on sèche 48 heures à 150°C. On obtient un catalyseur contenant 3,25% d'oxyde de Re déposé sur alumine activée. Le catalyseur B peut aussi être préparé par la sulfuration d'un sel, thiosel ou autre composé du Re, tel que thiorhénate d'ammonium, fixé sur un support d'alumine, de kieselguhr ou de charbon actif. Le catalyseur C est préparé comme décrit dans le brevet belge N° 874 616 (catalyseur HARSHAW Ni 0301 T dopé par 5% en poids d'hydroxyde de césium).

Les réactions sont mises en oeuvre dans un réacteur tubulaire en acier inoxydable de 45 cm de long et de 2 cm de diamètre. On introduit, pour chacun des essais 83,5 g de catalyseur, correspondant à 0,1 litre.

Avant la réaction, les catalyseurs sont sulfurés à 370°C pendant 5 à 6 heures par l'hydrogène sulfuré.

Les réactifs $CO/H_2S/H_2$ sont alors introduits dans un rapport 1/4/4 après mélange et préchauffage à 278°C sous une pression de 30 bars, avant d'atteindre le réacteur tubulaire chargé de catalyseur. La température à l'intérieur du réacteur est maintenue à 295°C. La vitesse spatiale des gaz, exprimée par rapport au monoxyde de carbone est de 50 litres de CO par heure et par litre de catalyseur. Le mélange sortant du réacteur est ensuite détendu à la pression atmosphérique par l'intermédiaire d'un régulateur de pression amont et analysé par chromatographie en phase vapeur.

Les résultats après une passe à travers le réacteur sont exprimés en pourcentage de monoxyde de carbone transformé et en sélectivité en différents produits par rapport au CO transformé. Les conversions et sélectivités (les définitions sont données dans le Nota page 18) sont fournies dans le tableau suivant dans des conditions de réaction identiques pour les 3 catalyseurs A, B et C.

On constate que la sélectivité en méthyl-mercaptan est la plus élevée pour le catalyseur A, tandis que très peu de sous-produits (tels que méthane, diméthyl-sulfure de carbone) sont détectés.

**Tableau 1**

| Cataly- seur | Conversion du CO (% mole) | Sélectivité (% mole) | | | |
|---|---|---|---|---|---|
| | | $CH_3SH$ | $CO_2$ | $CH_4$ | $DMS+CS_2$ |
| A ($K_2WO_4$ Alumine) | 75,8 | 55,2 | 44,1 | 0,7 | – |
| B ($Re_2O_7$ Alumine) | 92,0 | 47,2 | 41,0 | 7,4 | 4,4 |
| C (NiO+ CsOH Alumine) | 91,4 | 50,6 | 45,4 | 3,7 | 0,3 |

On peut constater que ces résultats sont nettement meilleurs que ceux de l'art antérieur, pour une vitesse spatiale de 50h$^{-1}$ (comparer avec USP 4 410 731, col. 3, tableau 1, run 6).

**Exemple 2**

Les trois catalyseurs A à C sont essayés dans la réaction faisant intervenir le mélange réactionnel $CO_2$, $H_2S$, $H_2$ dans le rapport 1/4/4, à une vitesse spatiale égale à 54 litres de $CO_2$ par litre de catalyseur et par heure. La température du préchauffeur est maintenue à 270°C et celle de la réaction catalytique à 295°C.

Les résultats exprimés en conversion par passe du $CO_2$ et en sélectivité en différents produits par rapport au $CO_2$, sont fournis dans le Tableau 2 suivant.

**Tableau 2**

| Cataly- seur | Conversion du $CO_2$ (% mole ) | Sélectivité (% mole) | | | |
|---|---|---|---|---|---|
| | | $CH_3SH$ | $CO$ | $CH_4$ | $DMS+CS_2$ |
| A | 21,0 | 51,1 | 48,9 | – | – |
| B | 28,0 | 64,6 | 25,2 | 7,9 | 2,3 |
| C | 19,7 | 46,5 | 50,9 | 2,6 | – |

Les catalyseurs A et B sont dans le cas de la réaction ($CO_2$, $H_2S$, $H_2$) plus adaptés que le catalyseur C de l'art antérieur. En particulier, le catalyseur A ne conduit pas à la formation de sous-produit gênant.

**Exemple 3**

Les opérations de l'exemple 2 sont répétées, mais avec recyclage des gaz sortant du réacteur. La vitesse d'arrivée des gaz dans le réacteur reste de 54 litres de $CO_2$ par litre de catalyseur et par heure dans un rapport $CO_2/H_2S/H_2$ de 1/4/4, mais on règle la vitesse spatiale des gaz dans le réacteur par une pompe à recyclage chauffée, après avoir éliminé le méthyl-mercaptan par fractionnement et l'eau résiduelle par passage de l'effluent de sortie du réacteur sur un tamis moléculaire. Les gaz ne contiennent plus que 0,1% d'eau. La vitesse spatiale des gaz, imposée par la pompe à recyclage, est équivalente à 1 760 litres de $CO_2$ par litre de catalyseur et par heure.

Le Tableau 3 donne les résultats obtenus avec le recyclage des gaz.

Comme déjà mentionné plus haut, les définitions des termes "conversion et sélectivité" se trouvent dans le Nota à la fin de la page 10.

**Tableau 3**

| Catalyseur | Conversion du $CO_2$ (% mole) | Sélectivité (% mole) | | | | |
|---|---|---|---|---|---|---|
| | | $CH_3SH$ | CO | COS | $CH_4$ | $DMS + CS_2$ |
| A | 86 | 82 | 15 | 3 | – | – |
| B | 93 | 85 | 6 | 2 | 3 | 4 |
| C | 84 | 78 | 18 | 2 | 2 | – |

Ainsi, le procédé de l'invention conduit-il, avec le catalyseur A à une production horaire de méthyl-mercaptan de

54 x 0,86 x 0,82 = 38 litres

alors que les meilleurs résultats du Tableau 5 dans l'US 4 410 731 (deux dernières lignes) correspondent à:

5 x 0,52 ; 2,6 litres et

60 x 0,24 ; 14,4 litres.

On voit que l'amélioration due à la présente invention est considérable; elle est obtenue grâce à l'abaissement de la teneur en eau dans les gaz traversant le réacteur, du fait de l'injection des gaz recyclés, secs, à une grande vitesse spatiale.

**Nota**

Dans la littérature technique américaine le terme "conversion" désigne ce que l'on nomme "rendement" en France, c'est-à-dire le nombre de moles de CO ou $CO_2$ sur 108 moles initiales, réellement transformées en méthyl-mercaptan. Par contre, "conversion" signifie en France le % des moles initiales consommées par la réaction tant pour faire du méthyl-mercaptan que des produits secondaires. La part % ayant donné naissance au mercaptan seul s'appelle "sélectivité".

Donc : rendement = conversion x sélectivité (France)

rendement = "conversion to MM" (USA)

d'où les différences entre les calculs donnés plus haut pour l'art antérieur et ceux de l'exemple 3.

**Revendications**

1. Perfectionnement au procédé de fabrication de méthylmercaptan par chauffage d'un gaz renfermant du CO ou/et $CO_2$, $H_2S$ et $H_2$ au contact d'un catalyseur à base d'un sulfure de métal lourd, avec recyclage de la fraction de gaz n'ayant pas réagi, caractérisé en ce que le gaz à recycler est débarrassé de l'eau qu'il contient à la suite de la réaction au contact du catalyseur.

2. Procédé suivant la revendication 1, caractérisé en ce que le recyclage est effectué avec une vitesse spatiale d'environ 50 à 2 000 litres de CO ou/et $CO_2$ par heure, par litre d'espace catalytique, et plus particulièrement 500 à 1800 litres.

3. Procédé suivant la revendication 1 ou 2, caractérisé en ce que dans le mélange gazeux employé, les rapports molaires des constituants (CO ou/et $CO_2$)/$H_2S$/$H_2$ sont compris entre 1/3/3 et 1/4/6 et, de préférence, très proches ou égaux à 1/4/4.

4. Procédé suivant une des revendications 1 à 3, caractérisé en ce que le catalyseur est un sulfure de tungstène employé seul, sans autres sulfures de métaux lourds, sur support d'alumine activée.

5. Procédé suivant la revendication 4, caractérisé en ce que le sulfure de tungstène provient de la sulfuration à l'$H_2S$ d'un tungstate de métal alcalin supporté par de l'alumine activée, le produit avant sulfuration ayant ete de préférence calciné vers 450° dans une atmosphère d'air.

6. Procédé suivant une des revendications 1 à 3, caractérisé en ce que le catalyseur est un sulfure de rhénium, de préférence préparé par sulfuration d'un sel, thiosel ou autre composé du Re, en particulier du perrhénate ou thiorhénate d'ammonium sur un support d'alumine, de kieselguhr ou de charbon actif, dans une atmosphère de $H_2S$.

7. Procédé suivant une des revendications précédentes, caractérisé en ce qu'après la séparation du méthylmercaptan du gaz passé sur le catalyseur, le gaz est soumis à la dessiccation avant d'êre envoyé à nouveau au contact du catalyseur.

8. Procédé suivant la revendication 7, caractérisé en ce que la dessiccation est réalisée par passage à travers un tamis moléculaire.

## Patentansprüche

1. Verbesserung des Verfahrens zur Herstellung von Methylmercaptan durch Erhitzen eines Gases mit einem Gehalt an CO oder/und $CO_2$, $H_2S$ und $H_2$ im Kontakt mit einem Katalysator auf der Basis eines Schwermetallsulfids unter Rückführung der nicht-umgesetzten Gasfraktion, dadurch gekennzeichnet, dass das zurückzuführende Gas von dem Wasser, das es als Folge der Reaktion im Kontakt mit dem Katalysator enthält, befreit wird.

2. Verfahren nach Anspruch 1, dadurch gekennzeichnet, dass die Rückführung mit einer Raumgeschwindigkeit von etwa 50 bis 2000 Liter CO oder/und $CO_2$ pro Stunde pro Liter Katalysatorraum und insbesondere von 500 bis 1800 Liter durchgeführt wird.

3. Verfahren nach Anspruch 1 oder 2, dadurch gekennzeichnet, dass im verwendeten Gasgemisch die Molverhältnisse der Bestandteile (CO oder/und $CO_2$)/$H_2S$/$H_2$ 1/3/3 bis 1/4/6 betragen und vorzugsweise sehr nahe oder gleich den Werten 1/4/4 sind.

4. Verfahren nach einem der Ansprüche 1 bis 3, dadurch gekennzeichnet, dass es sich beim Katalysator um ein Wolframsulfid, das allein - ohne andere Schwermetallsulfideauf aktiviertem Aluminiumoxid als Träger verwendet wird, handelt.

5. Verfahren nach Anspruch 4, dadurch gekennzeichnet, dass das Wolframsulfid aus der Schwefelung mit $H_2S$ eines auf aktiviertes Aluminiumoxid als Träger aufgebrachten Alkalimetallwolframats stammt, wobei das Produkt vor der Schwefelung vorzugsweise in einer Luftatmosphäre bis 450°C calciniert worden ist.

6. Verfahren nach einem der Ansprüche 1 bis 3, dadurch gekennzeichnet, dass es sich beim Katalysator um ein Rheniumsulfid handelt, das vorzugsweise durch Schwefelung eines Salzes, Thiosalzes oder einer anderen Verbindung von Re, insbesondere von Ammoniumperrhénat oder Ammoniumthiorhenat auf Aluminiumoxid, Kieselgur oder Aktivkohle als Träger in einer $H_2S$-Atmosphäre hergestellt worden ist.

7. Verfahren nach einem der vorstehenden Ansprüche, dadurch gekennzeichnet, dass nach der Abtrennung des Methylmercaptans aus dem Gas, das den Katalysator passiert hat, das Gas einer Trocknung unterworfen wird, bevor es in erneuten Kontakt mit dem Katalysator gebracht wird.

8. Verfahren nach Anspruch 7, dadurch gekennzeichnet, dass die Trocknung durch Passage über ein Molekularsieb vorgenommen wird.

## Claims

1. Improvement in the process of manufacture of methyl-mercaptan by heating a gas containing CO and/or $CO_2$, $H_2S$ and $H_2$ in contact with a heavy metal sulphide catalyst, with recycling of the non-reacted gas fraction, characterised in that the gas to be recycled is freed from the water which it contains following the reaction in contact with the catalyst.

2. Process according to claim 1, characterised in that the recycling is effected at a spatial velocity of about 50 to 2000 litres of CO and/or $CO_2$ per hour per litre of catalytic volume and more particularly at 500 to 1800 litres.

3. Process according to claim 1 or 2, characterised in that in the gaseous mixture employed the molar ratios of the constituents (CO and/or $CO_2$)/$H_2S$/$H_2$ are from 1/3/3 to 1/4/6 and preferably are close or equal to 1/4/4.

4. Process according to any of claims 1 to 3, characterised in that the catalyst is a sulphide of tungsten employed alone, without other heavy metal sulphides, on an activated alumina support.

6

5. Process according to claim 4, characterised in that the tungsten sulphide is derived from the sulphuration with $H_2S$ of an alkali metal tungstate supported on the activated alumina, the product before sulphuration preferably having been calcined at about 450° in an air atmosphere.

6. Process according to any of claims 1 to 3, characterised in that the catalyst is a rhenium sulphide, preferably prepared by the sulphuration of a salt, thiosalt or other compound of Re, in particular ammonium perrhenate or thiorhenate on an alumina, kieselguhr or active carbon support in an $H_2S$ atmosphere.

7. Process according to any of the preceding claims, characterised in that after separation of the methyl-mercaptan from the gas passed over the catalyst, the gas is subjected to desiccation before being again brought into contact with the catalyst.

8. Process according to claim 7, characterised in that the desiccation is effected by passage through a molecular sieve.